# EUROPÄISCHE PATENTSCHRIFT

(11) **EP 0 510 715 B1**
(45) Veröffentlichungstag und Bekanntmachung des Hinweises auf die Patenterteilung: **30.10.1996**
(21) Anmeldenummer: 92107155.1
(22) Anmeldetag: 27.04.1992
(51) Int. Cl.: A61F 13/15

(54) **Verfahren zum Anbringen von elastischen Elementen um Beinöffnungen von Wegwerfbekleidungsstücken**
Process for attaching elastic elements around the leg openings of disposable clothing
Procédé pour l'attachement d'éléments élastiques autour des ouvertures de jambe des vêtements à jeter

(30) Priorität: 25.04.1991 JP 122429/91
(43) Veröffentlichungstag der Anmeldung: 28.10.1992
(73) Patentinhaber: UNI-CHARM CORPORATION, Kawanoe-shi Ehime-ken (JP)
(72) Erfinder: Nomura, Hironori, Ehime-ken (JP); Shimakawa, Taiji, Kanonji-shi, Kagawa-ken (JP); Matsura, Yoshinori, Nakatado-gun, Kagawa-ken (JP); Yamamoto, Hiroki, Mitoyo-gun, Kagawa-ken (JP); Ohnishi, Hirofumi, Ehime-ken (JP)
(74) Vertreter: Sperling, Rüdiger, Dipl.-Ing.

(56) Entgegenhaltungen:
- EP-A- 0 048 011
- EP-A- 0 405 575
- EP-A- 0 417 766
- GB-A- 2 196 834

## Beschreibung

Diese Erfindung betrifft ein Verfahren zum Anbringen von elastischen Elementen um Beinöffnungen von Wegwerfkleidungsstücken, wie etwa Windeln, Erziehungshöschen oder Inkontinenzhöschen.

Aus der EP-A-0 048 011 ist ein Verfahren zum kontinuierlichen Herstellen von Wegwerfwindeln bekannt, bei dem nach dem Aufbringen und Ankleben von sinusförmigem elastischem Band auf einer Grundlage und entsprechendem aufeinanderfolgenden Aufbau der Windelkonstruktion die Bahn zur Erstellung von Einzelkleidungsstücken an den jeweiligen Umkehrpunkten der Sinusamplituden voneinander getrennt sind.

Beim Klebevorgang wird, soweit es in dieser Schrift das Ausführungsbeispiel gemäß Fig. 5 betrifft, das elastische Band nicht über den gesamten Bereich angeklebt, sondern es werden Bereiche im Kreuzungspunkt freigelassen. Der Kreuzungspunkt befindet sich bei diesen Windein im Schritt. Aufgrund des Umstands, daß das elastische Band fortlaufend auch über den Schrittbereich gezogen wird, entsteht ein höherer Materialbedarf an elastischem Band.

Zum Anbringen von elastischen Elementen um die Beinöffnungen von Wegwerfkleidungsstücken wurden bereits verschiedene Verfahren vorgeschlagen, von denen einige auch in die Praxis umgesetzt wurden. In der EP 0405575 haben die Erfinder ebenfalls ein zum Anbringen von elastischen Elementen um Beinöffnungen von Wegwerfkleidungsstücken, wie etwa Erziehungshöschen, geeignetes Verfahren vorgeschlagen, bei dem die elastischen Elemente in Bögen verlaufen, so daß die Kleidungsstücke einen guten Sitz um das Bein des Trägers haben.

Bei den nach dem Verfahren gemäß dem Stand der Technik hergestellten Höschen tritt jedoch das Problem auf, daß zwischen den beiden Beinöffnungen ein nutzloser Abschnitt der elastischen Elemente übrigbleibt. Insbesondere bei Verwendung von Vliesstoffen oder Kunststoffolien, die recht durchscheinend sind, als Material für die obere und untere Lage der Kleidungsstücke ist der nutzlose Abschnitt durch diese Lagen sichtbar, was nicht nur die Kleidungsstücke unattraktiv macht, sondern auch die Herstellungskosten erhöht, da die Menge der in jedem Kleidungsstück verwendeten elastischen Elemente um die dem nutzlosen Abschnitt entsprechende Menge erhöht wird. Zwar ist es möglich, während der Herstellung den nutzlosen Abschnitt der elastischen Elemente abzutrennen, womit jedoch weiterhin die dem nutzlosen Abschnitt entsprechende Materialmenge zusätzlich benötigt wird.

Demgemäß ist es eine Aufgabe der vorliegenden Erfindung, ein Verfahren zum Anbringen von elastischen Elementen um Beinöffnungen von Wegwerfkleidungsstücken aufzuzeigen, bei dem grundsätzlich das Verfahren nach dem Stand der Technik angewandt wird, jedoch nicht die vorstehend erwähnten Probleme auftreten, die bei diesem bekannten Verfahren vorkommen.

Es ist eine weitere Aufgabe der Erfindung, ein Verfahren zum Anbringen von elastischen Elementen um Beinöffnungen von Wegwerfkleidungsstücken aufzuzeigen, durch das die Zugspannung des elastischen Elements für die jeweilige Beinöffnung im wesentlichen auf die obere Hälfte der Beinöffnung verteilt wird.

### BESCHREIBUNG DER ERFINDUNG

Erfindungsgemäß werden die vorstehend dargelegten Aufgaben durch ein Verfahren gelöst, das sowohl bei Kleidungsstücken mit offener Taillenöffnung als auch bei Kleidungsstücken mit geschlossener Taillenöffnung anwendbar ist und im wesentlichen folgende Schritte umfasst:

Das Bilden einer Basisbahn durch kontinuierliches Zuführen und Verbinden mit Klebstoff von ersten und zweiten endlosen elastischen Elementen in gestrecktem Zustand auf eine Oberfläche einer in Bewegung befindlichen ersten Bahn in der Weise, daß die elastischen Elemente sinuskurvenartige Kurven beschreiben, deren Scheitelpunkte und tiefste Punkte einander symmetrisch gegenüberliegend angeordnet sind und die einander an ihren Enden überkreuzen, so daß ringförmige elastische Bereiche gebildet werden; das Bilden einer ersten Verbundbahn mittels Durchtrennen der Basisbahn entlang Linien, die jeweils quer durch die jeweiligen Kreuzungspunkte verlaufen, an welchen sich die ersten und zweiten elastischen Elemente überkreuzen, und punktweisem Verkleben der jeweiligen abgetrennten Streifen mittels Klebstoff auf einer zweiten in Bewegung befindlichen Bahn entlang deren in Längsrichtung sich erstreckendem Mittelbereich; das Bilden einer zweiten Verbundbahn durch Verkleben einer dritten in Bewegung befindlichen Bahn mittels Klebstoff auf einer Oberfläche der ersten Verbundbahn; das Bilden zusammenhängender Kleidungsstückzuschnitte durch Ausschneiden von Bereichen der zweiten Verbundbahn, die von den jeweiligen ringförmigen elastischen Bereichen umgeben sind, um Ausnehmungen für die Beinöffnungen zu bilden; und das Herstellen einzelner Kleidungsstücke mittels Abtrennen der zusammenhängenden Kleidungsstückzuschnitte entlang von Linien, die jeweils in Längsrichtung der zweiten Verbundbahn einen zugehörigen ringförmigen elastischen Bereich halbieren.

Bei Kleidungsstücken, die an der Taillenöffnung geschlossen sind, werden die zusammenhängenden Kleidungsstückzuschnitte durch Übereinanderfalten der zweiten Verbundbahn in Querrichtung entlang ihrer in Längsrichtung verlaufenden Mittellinie umgeformt, worauf die gefaltete zweite Verbundbahn entlang den Linien, die die jeweiligen elastischen Bereiche halbieren, mit quer verlaufenden Siegellinien versehen wird und so die einzelnen Kleidungsstücke gebildet werden. Diese einzelnen Kleidungsstücke werden von den zusammenhängenden Kleidungsstückzuschnitten so abgetrennt, daß die Siegellinien erhalten bleiben, die dadurch die Seitenränder der jeweiligen einzelnen Kleidungsstücke bilden.

Normalerweise umfaßt die zweite Bahn eine flüssigkeitsdurchlässige Lage und die dritte Bahn eine flüssigkeitsundurchlässige Lage. Um Kleidungsstücke mit flüssigkeitsabsorbierendem Kern zu erhalten, werden die flüssigkeitsabsorbierenden Kerne jeweils zwischen zwei benachbarten ringförmigen elastischen Bereichen auf der ersten Verbundbahn angeordnet, bevor die dritte Bahn auf die erste Verbundbahn aufgelegt wird.

Diese und weitere Merkmale und Vorteile der Erfindung sind aus der nachfolgenden Beschreibung mit Bezug auf die beiliegenden Zeichnungen besser zu verstehen.

### KURZE BESCHREIBUNG DER ZEICHNUNG

- Fig. 1: zeigt eine isometrische Darstellung eines Kleidungsstückes, das durch ein Verfahren gemäß der Erfindung herzustellen ist;
- Fig. 2: ist eine auseinandergezogene isometrische Darstellung des Kleidungsstückes;
- Fig. 3: ist eine schematische isometrische Darstellung einer Traversiereinrichtung, die zum Anbringen von ersten und zweiten endlosen elastischen Elementen an einer ersten Bahn dient;
- Fig. 4: ist eine Draufsicht auf eine Basisbahn, die die erste Bahn und erste und zweite endlose elastische Elemente aufweist, die durch die Traversiereinrichtung aufgebracht wurden;
- Fig. 5: ist eine Draufsicht auf eine Anordnung, die eine zweite Bahn, von der Basisbahn abgetrennte Streifen, die punktweise auf die zweite Bahn geklebt sind, um so eine Verbundbahn zu bilden, sowie einen Kern und elastische Elemente für die Taillenöffnung umfaßt, die auf der Oberfläche der ersten Verbundbahn angebracht sind;
- Fig. 6: ist eine Draufsicht auf eine Anordnung, die die erste Verbundbahn und eine mit der ersten Verbundbahn verklebte dritte Bahn umfaßt, so daß eine zweite Verbundbahn gebildet ist, wobei die vorgesehenen Abschnitte der zweiten Verbundbahn ausgeschnitten wurden, um so die Ausnehmungen für die jeweiligen Beinöffnungen zu bilden; und
- Fig. 7: ist eine Draufsicht auf einen Zustand, in dem die zweite Verbundbahn an gewünschten Stellen mit Siegellinien versehen wurde, um die einzelnen Kleidungsstücke zu begrenzen, wobei ein einzelnes Kleidungsstück von der zweiten Verbundbahn abgetrennt wurde.

### BEVORZUGTE AUSFÜHRUNGSFORMEN DER ERFINDUNG

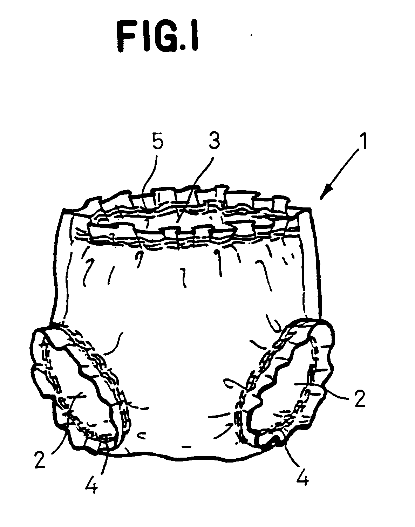

Fig. 1 ist eine isometrische Darstellung und zeigt ein Beispiel eines Kleidungsstückes, das durch ein erfindungsgemäßes Verfahren hergestellt wurde. Ein allgemein mit 1 bezeichnetes Kleidungsstück ist mit Beinöffnungen 2 und einer Taillen-(Rumpf-)öffnung 3 versehen, die mit elastischen Elementen 4 bzw. 5 ausgerüstet sind.

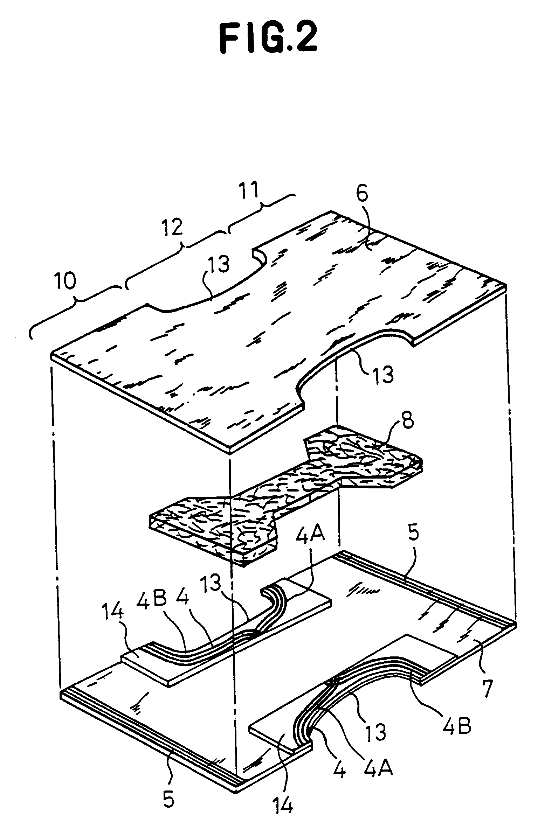

Fig. 2 ist eine auseinandergezogene isometrische Darstellung des Kleidungsstückes 1. Wie hier gezeigt ist, umfaßt das Kleidungsstück 1 eine aus sowohl in der Länge als auch in der Breite dehnbarem, flüssigkeitsdurchlässigem Vliesstoff hergestellte obere Lage 6, eine aus sowohl in der Länge als auch in der Breite dehnbarem, jedoch flüssigkeitsundurchlässigem Vliesstoff hergestellte untere Lage 7, einen matten- oder plattenartigen Flüssigkeitsabsorptionskern 8, der hauptsächlich aus Faserpulpe hergestellt ist, und die um die Beinöffnungen bzw. die Taillenöffnung angeordneten elastischen Elemente 4 bzw. 5. Ein zwischen einem Vorderteil 10 und einem Hinterteil 11 der oberen und unteren Lage 6, 7 angeordneter Schrittbereich 12 weist an seinen gegenüberliegenden Seitenrändern identische Ausnehmungen 13 auf, die die Beinlöcher 2 begrenzen. Wie in der Fig. nicht dargestellt, kann die untere Lage 7 auch einen sowohl in der Länge als auch in der Breite dehnbaren und flüssigkeitsdurchlässigen Vliesstoff umfassen, jedoch zusätzlich noch mit einer ebenfalls sowohl in der Länge als auch in der Breite dehnbaren, aber flüssigkeitsundurchlässigen Kunststoffolie versehen sein, die mittels Klebstoff punktweise mit einer Innenfläche desselben verklebt ist. Durch eine solche alternative Anordnung können flüssige Exkremente sicher am Durchdringen der unteren Lage 7 gehindert werden. Darüber hinaus wird die Dehnbarkeit und damit auch die Dehnspannung der oberen und unteren Lagen 6, 7 als den Hauptbestandteilen des Kleidungsstückes 1 verbessert und damit die Paßform des Kleidungsstückes 1 am Körper des Trägers weiter verbessert, wenn die Folie mittels Klebstoff punktweise wenigstens entlang des äußeren Umfanges mit der oberen Lage 6 verklebt ist.

Die um die jeweiligen Beinöffnungen vorgesehenen elastischen Elemente 4 sind auf der Oberfläche der unteren Lage 7 entlang den einander gegenüberliegenden Ausnehmungen 13 angebracht, wobei relativ dünne, sowohl in der Länge als auch in der Breite dehnbare Basisstreifen 14 (oder Streifen 17', wie weiter unten erläutert wird) zwischengelegt sind, die jeweils hydrophobe Folie oder wasserabweisenden Vliesstoff oder Kunststoffolie umfassen. Das elastische Element 4 umfaßt elastische Einzelelemente 4A, 4B, die sich an der in Längsrichtung gesehenen Mittellinie der zugehörigen Ausnehmung 13 überkreuzen. Die elastischen Einzelelemente 4A, 4B umfassen jeweils mehrere fadenförmige Grundelemente aus Naturkautschuk oder synthetischem Gummi. Das elastische Element 5 umfaßt ebenfalls mehrere fadenförmige Grundelemente, die um die Taillenöffnung an der unteren Lage 7 angebracht sind.

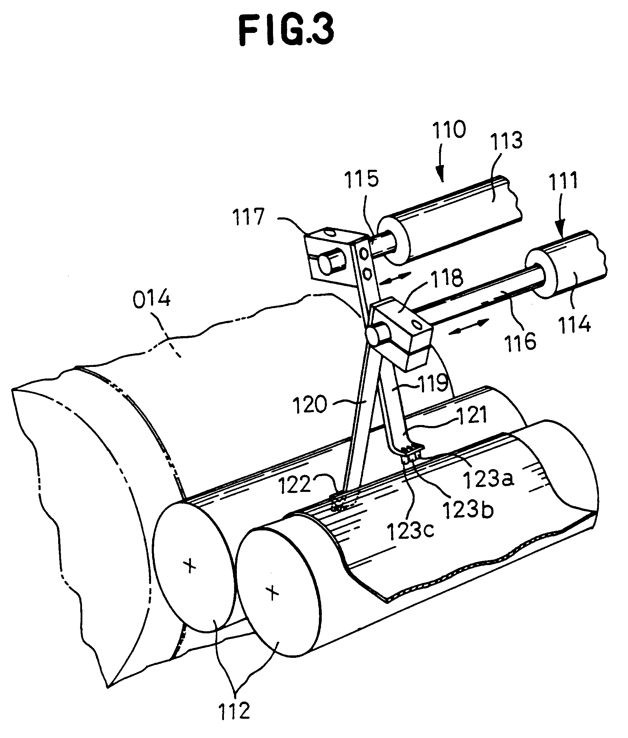

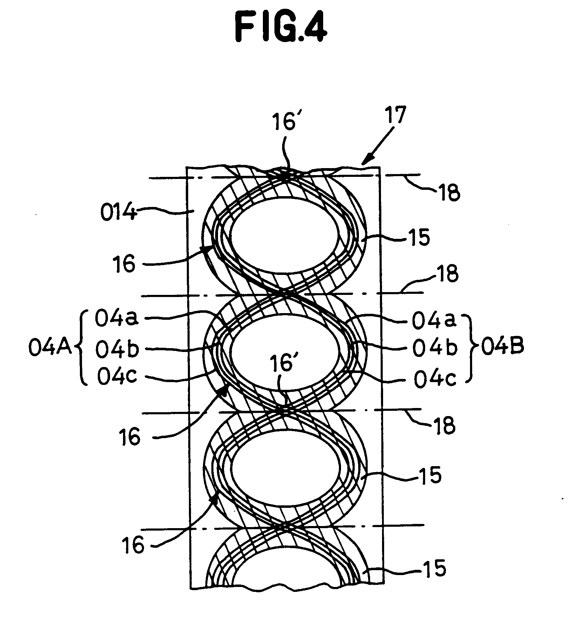

Fig. 3 ist eine isometrische schematische Darstellung von zwei Traversiereinrichtungen 110, 111, die zum Anbringen von ersten und zweiten endlosen elastischen Elementen, aus welchen die einzelnen elastischen Einzelelemente jeweils gebildet werden, an einer ersten Bahn 014 verwendet werden, aus der in der vorstehend erwähnten Weise einzelne Basisstreifen gebildet werden. Fig. 4 ist eine Draufsicht, die den Zustand zeigt, in dem erste und zweite endlose elastische Elemente 04A, 04B auf der ersten Bahn 014 angebracht wurden. Ein derartiges Anbringungsverfahren ist mit Ausnahme der Verwendung der Traversiereinrichtung bekannt und kann unter Verwendung herkömmlicher Geräte zur Herstellung von Wegwerfwindeln, wie beispielsweise in der EP 0405575 der Anmelderin der vorliegenden Erfindung beschrieben.

In Fig. 3 sind zwei Traversiereinrichtungen 110, 111 gezeigt, die zylindrische Halter 113, 114 umfassen, die nahe an und parallel zu einem Klemmwalzenpaar 112 angeordnet sind und in die jeweils verschiebliche Stangen 115, 116 eingesetzt sind. An den freien Enden der Stangen sind jeweils Halteblöcke 117, 118 befestigt, von denen Führungsstäbe 119, 120 jeweils herabhängen. Die Führungsstäbe 119, 120 sind an ihren umgebogenen Enden jeweils mit kleinen zylindrischen Führungen 123a, 123b, 123c versehen. Diese Führungen 123a, 123b, 123c sind quer zur ersten Bahn 014 zueinander beabstandet angeordnet. Die umgebogenen Enden 121, 122 sind den Umfangsflächen der zugehörigen Klemmwalzen 112 benachbart angeordnet. Die verschieblichen Stangen 115, 116 werden von jeweiligen Gleitnuten-Traversiermechanismen (nicht dargestellt) gesteuert, die mit den Basisenden dieser verschieblichen Stangen funktionell in Verbindung stehen.

Wie Fig. 4 zeigt, wird die erste Bahn 014 entlang ihrem in Längsrichtung verlaufenden Mittelbereich mit Klebstoff beschichtet, während die erste Bahn 014 mit vorgegebener Geschwindigkeit in Längsrichtung bewegt wird, so daß in Längsrichtung der Bahn endlose elliptische Klebebereiche 15 gebildet werden können.

In die Führungen 123a, 123b, 123c der Traversiereinrichtung 110 bzw. 111 werden jeweils fadenförmige Grundelemente 04a, 04b, 04c, die das erste und zweite endlose elastische Element 04A, 04B bilden, eingeführt, wobei sie mit einem bestimmten Prozentsatz gedehnt gehalten werden. Zugleich werden die Führungsstäbe 119, 120 mit vorgegebener Geschwindigkeit quer über die erste Bahn 014 hin und her bewegt, so daß die fadenförmigen Grundelemente 04a, 04b, 04c, die das erste bzw. zweite endlose elastische Element 04A, 04B bilden, auf den zugehörigen Klebebereichen 15 sinusförmige Kurven beschreiben. Genauer gesagt werden die fadenförmigen Grundelemente so geführt, daß die Scheitelpunkte wie auch die tiefsten Punkte der jeweiligen Kurven, die von den ersten und zweiten endlosen elastischen Elementen 04A, 04B beschrieben werden, einander symmetrisch gegenüberliegend angeordnet sind und sich an ihren Enden überkreuzen, so daß die ringförmigen elastischen Bereiche 16 gebildet werden, die wiederum zur Bildung einer endlosen Basisbahn 17 durch die Klemmwalzen 112 zwangsweise mit den jeweiligen Klebebereichen 15 verbunden werden.

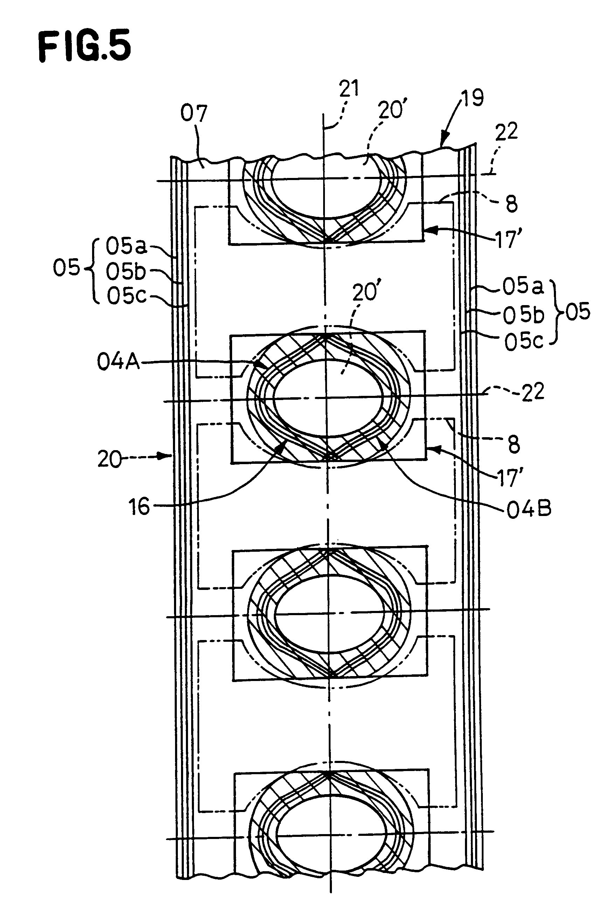

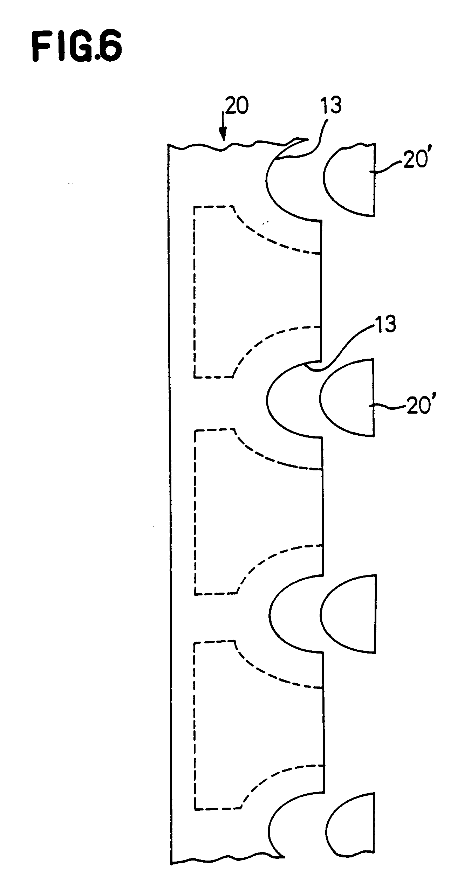

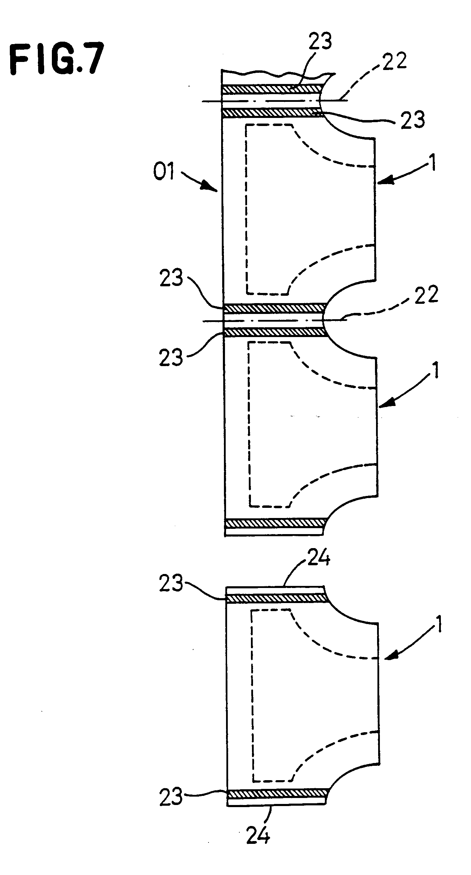

Fig. 5 ist eine Draufsicht auf eine erste Verbundbahn 19, die eine zweite Bahn 07 als Ausgangsmaterial der unteren Lage 7 umfaßt, die mit den Streifen 17' versehen ist, die durch Abtrennen von der Basisbahn 17 erhalten werden und punktweise mittels Klebstoff auf die zweite Bahn 07 aufgeklebt werden. Die Betriebsabläufe für das Schneiden und Verkleben sind beispielsweise unter Verwendung der von der Erfinderin in der EP 0304044 aufgezeigten Vorrichtung durchführbar. Fig. 6 zeigt eine Draufsicht auf eine zweite Verbundbahn 20, die zur Bildung der Ausnehmungen 13 für die jeweiligen Beinöffnungen teilweise ausgeschnitten ist. Fig. 7 zeigt eine Draufsicht auf zusammenhängende Kleidungsstückzuschnitte 01, die an bestimmten Stellen mit Siegellinien 23 versehen sind, die das einzelne Kleidungsstück 1 begrenzen.

Wie Fig. 5 zeigt, wird die Basisbahn 17 jeweils entlang Linien 18 abgetrennt, die quer durch Kreuzungspunkte 16' verlaufen, an denen sich die ersten und zweiten endlosen elastischen Elemente 04A, 04B überkreuzen und so die einzelnen ringförmigen elastischen Bereiche 16 bilden, wie in Fig. 4 dargestellt. Die jeweiligen abgetrennten Streifen 17' werden punktweise mittels Klebstoff auf eine in Bewegung befindliche zweite Bahn 07 entlang ihrem in Längsrichtung verlaufenden Mittelbereich aufgeklebt. Gleichzeitig werden fadenförmige Grundelemente 5a, 5b, 5c, die gemeinsam ein elastisches Element 05 für die Taillenöffnung bilden, mittels Klebstoff in Längsrichtung entlang den gegenüberliegenden Seitenrändern der zweiten Bahn 07 aufgeklebt, um so die erste Verbundbahn 19 zu bilden.

Der Kern 8, der bereits in Sanduhrform vorgeformt ist, wird zwischen je zwei benachbarten ringförmigen elastischen Bereichen 16 angeordnet, worauf eine nicht dargestellte dritte Bahn als Ausgangsmaterial für die obere Lage 6 mittels Klebstoff auf die Oberfläche der ersten Verbundbahn 19 aufgeklebt wird, um so die zweite Verbundbahn 20 zu bilden.

Die zweite Verbundbahn 20 wird entlang ihrer in Längsrichtung verlaufenden Mittellinie 21 quer übereinandergefaltet.

Wie Fig. 6 zeigt, werden Abschnitte 20' der zweiten Verbundbahn 20, die jeweils von den ringförmigen elastischen Bereichen 16, die in Fig. 5 gezeigt sind, umgeben sind, ausgeschnitten, um so die Ausnehmungen 13 für die Beinöffnungen zu bilden.

Wie Fig. 7 zeigt, wird die Verbundbahn 20 in Längsrichtung beiderseits einer in Fig. 5 dargestellten Linie 22, die jeweils die ringförmigen elastischen Bereiche 16 halbiert, mit je einer quer verlaufenden Heißsiegellinie bzw. Schallschweißlinie 23 versehen, wodurch die einzelnen Kleidungsstücke 1 begrenzt werden und der zusammenhängende Kleidungsstückzuschnitt 01 gebildet wird.

Das einzelne Kleidungsstück 1 wird entlang der Linie 22 vom zusammenhängenden Kleidungsstückzuschnitt 01 abgetrennt, so daß zwei Siegellinien 23 an jedem einzelnen Kleidungsstück 1 verbleiben und gegenüberliegende Seitenränder 24 dieses Kleidungsstückes 1 bilden. Dadurch wird das in Fig. 1 gezeigte einzelne Kleidungsstück 1 erhalten.

Das Bilden der Ausnehmungen 13 in der zweiten Verbundbahn 20, d.h. das Ausschneiden der Abschnitte 20', kann vor oder nach dem Falten der zweiten Verbundbahn 20 oder nach dem Bilden der Siegellinien 23 erfolgen. Handelt es sich um ein an der Taillenöffnung offenes Kleidungsstück, so können die Siegellinien 23 durch eine Klebebandbefestigung ersetzt werden, die an gegenüberliegenden Seiten des Kleidungsstückes in dessem hinteren Bereich angebracht werden. Ist bei einem Kleidungsstück der Kern 8 nicht erforderlich, so wird selbstverständlich der Kern 8 nicht auf die erste Verbundbahn 19 aufgelegt.

Während die Erfindung so dargestellt und erläutert wurde, daß auf der ersten Bahn 014 die Klebebereiche 15 zum Anbringen der ersten und zweiten endlosen elastischen Elemente 04A, 04B auf der ersten Bahn 014 vorgesehen sind, ist es ebenso möglich, die Klebebeschichtung direkt auf die elastischen Elemente aufzubringen. Die Führungsstäbe 119, 120 können, wie in den Fig. nicht dargestellt, an ihren unteren Enden mit einer Klebstoffauftrageinrichtung versehen sein, die die Führungen 123a, 123b, 123c einschließt, und Klebstoff kann über einen flexiblen Schlauch unter vorgegebenem Druck von einer getrennt vorgesehenen Klebstoffzufuhrquelle der Klebstoffauftrageinrichtung zugeführt werden, so daß er auf die fadenförmigen Grundelemente, die die jeweiligen endlosen elastischen Elemente 04A, 04B bilden, in deren Längsrichtung aufgetragen werden kann. In diesem Fall kann die von der Anmelderin der vorliegenden Anmeldung in den US-Patenten Nr. 4.626.305 und 4.687.477 aufgezeigte Auftragvorrichtung (Düse) als Klebstoffauftragvorrichtung verwendet werden.

Erfindungsgemäß werden das erste und zweite endlose elastische Element 04A, 04B mittels Klebstoff auf die erste Bahn 014 aufgeklebt, wobei die elastischen Elemente von den Traversiereinrichtungen 110, 111 im wesentlichen jeweils in Sinuskurven gelegt werden, wie bereits erläutert wurde. Daher bestehen die elastischen Elemente 04A, 04B vorzugsweise aus den fadenförmigen Grundelementen 04a, 04b, 04c. Wären diese elastischen Elemente relativ breit, so könnten diese elastischen Elemente nicht ordnungsgemäß auf die Endlosbahn aufgeklebt werden und würden während des Klebevorganges leicht von der Bahn abgezogen, da sie bei Anwendung des erfindungsgemäßen Verfahrens gekrümmt bzw. gedreht werden sollten. Das erste und zweite endlose elastische Element 04A, 04B kann einen kreisförmigen (einschließlich elliptischen), rechteckigen oder anderen unbestimmten Querschnitt haben, wobei es jedoch nicht erwünscht ist, daß ein Verhältnis des größten Querschnittsmaßes zum kleinsten Querschnittsmaß übermäßig groß ist.

Das Anbringen des elastischen Elementes 05 für die Taillenöffnung an der ersten Verbundbahn 19 kann durch Anwendung der herkömmlichen Vorrichtungen und Verfahren zur Herstellung von Wegwerfwindeln durchgeführt werden. Dieses elastische Element 05 kann auch ein einzelnes bandförmiges Grundelement anstelle der vorstehend erwähnten Anzahl von fadenförmigen Grundelementen umfassen.

Gegenüber dem Verfahren nach dem Stand der Technik erlaubt das erfindungsgemäße Verfahren das effiziente Anordnen von elastischen Elementen um Beinöffnungen, ohne daß nutzlose Verbindungsabschnitte der elastischen Elemente zwischen den beiden Beinöffnungen vorliegen. Darüber hinaus ergibt das erfindungsgemäße Verfahren Kleidungsstücke, die im Bereich der Beinöffnungen verstärkt sind und in wirksamer Weise das Austreten von flüssigen Exkrementen um die Beinöffnungen verhindern können.

## Patentansprüche

1. Verfahren zum Herstellen von Kleidungsstücken (1), insbesondere Wegwerfkleidungsstücken mit elastischen Elementen um Beinöffnungen des Kleidungsstückes, gekennzeichnet durch die Schritte:
a) Bilden einer Basisbahn (17) durch kontinuierliches Zuführen und Verkleben mittels Klebstoff von ersten und zweiten, in gestrecktem Zustand gehaltenen endlosen elastischen Elementen (4A, 4B) auf eine Oberfläche einer in Bewegung befindlichen ersten Bahn (014) in der Weise, daß die elastischen Elemente sinuskurvenartige Kurven beschreiben, deren Scheitelpunkte und tiefste Punkte einander jeweils symmetrisch gegenüberliegend angeordnet sind und die einander an ihren *Wendepunkten* überkreuzen, so daß ringförmige elastische Bereiche gebildet werden;
b) Bilden einer ersten Verbundbahn (19) mittels Durchtrennen der Basisbahn (17) entlang Linien (18), die jeweils quer durch die jeweiligen Kreuzungspunkte (16') verlaufen, an welchen sich die ersten und zweiten elastischen Elemente (4A, 4B) überkreuzen, und punktweises Verkleben der jeweiligen abgetrennten Streifen mittels Klebstoff auf einer zweiten in Bewegung befindlichen Bahn (07) entlang deren in Längsrichtung sich erstreckendem Mittelbereich;
c) Bilden einer zweiten Verbundbahn (20) durch Verkleben einer dritten in Bewegung befindlichen Bahn mittels Klebstoff auf einer Oberfläche der ersten Verbundbahn (19);
d) Bilden zusammenhängender Kleidungsstückzuschnitte durch Ausschneiden von Bereichen der zweiten Verbundbahn (20), die von den jeweiligen ringförmigen elastischen Bereichen (16) umgeben sind, um Ausnehmungen (13) für die Beinöffnungen zu bilden; und
e) Herstellen einzelner Kleidungsstücke (1) mittels Abtrennen der zusammenhängenden Kleidungsstückzuschnitte entlang der Linien, die jeweils in Längsrichtung der zweiten Verbundbahn einen zugehörigen ringförmigen elastischen Bereich halbieren.

2. Verfahren nach Anspruch 1, **dadurch gekennzeichnet,** daß nach dem Verfahrensschritt c) in Anspruch 1 folgende Zwischenschritte ausgeführt werden:
f) Falten der zweiten Verbundbahn (20) in Querrichtung entlang ihrer in Längsrichtung verlaufenden Mittellinie (21), die die zweite Verbundbahn halbiert;
(g) Versehen der zweiten Verbundbahn (20) entlang Linien (22), die die jeweiligen ringförmigen Bereiche halbieren, mit Siegellinien (23), wodurch die einzelnen Kleidungsstücke begrenzt werden,
h) Abtrennen der einzelnen Kleidungsstücke (1) von dem zusammenhängenden Kleidungsstückzuschnitt (01) gleichzeitig mit oder nach Schritt (g), so daß die Siegellinien (23) erhalten bleiben und dadurch die Seitenränder (24) der einzelnen Kleidungsstücke bilden,
wobei der Verfahrensschritt (d) aus Anspruch 1 vor oder nach dem Schritt (f) durchgeführt werden kann.

## Claims

1. A method of making articles of clothing (1), especially disposable articles of clothing, with elastic elements around leg openings of the articles of clothing, characterized by the steps:
a) Forming a basic band (17) by continuously feeding first and second continuous elastic elements (4A, 4B) maintained in a stretched state and bonding them by means of an adhesive on a surface of a first band (014) in motion in such a manner that the elastic elements describe sinusoidal curves whose crests and troughs are arranged symmetrically opposite one another and which cross over each other at their points of inflection, so that ring-shaped elastic regions are formed;
b) Forming a first composite band (19) by cutting through the basic band (17) along lines (18) which each run transversely through the respective cross-over points (16) at which the first and second elastic elements (4A, 4B) cross over, and by bonding the respective cut off strips at points by means of an adhesive to a second band (07) in motion, along its central region extending in the longitudinal direction;
c) Forming a second composite band (20) by bonding a third band in motion by means of adhesive on a surface of the first composite band (19);
d) Forming attached clothing article sections by cutting out regions of the second composite band (20) which are surrounded by respective ring-shaped elastic regions (16), in order to form cut-outs (13) for the leg openings; and
e) Making a individual article of clothing (1) by separating the attached clothing article sections along the lines which each bisect in the longitudinal direction of the second composite band an associated ring-shaped elastic region.

2. A method according to claim 1, characterized in that the following intermediate steps are carried out after the method step c) in claim 1:
f) Folding the second composite band (20) in the transverse direction along its centre line (21) which runs in the longitudinal direction and bisects the second composite band;
g) Providing the second composite band (20) with sealed lines (23) along lines (22) which bisect the respective ring-shaped regions, whereby the individual articles of clothing are delimited,
h) Separating the individual articles of clothing (1) from the attached clothing article sections (01) simultaneously with or after step (g), so that the sealed lines (23) remain and thereby form the side edges (24) of the individual articles of clothing,
wherein the method step (d) of claim 1 can be carried out before or after step (f).

## Revendications

1. Procédé de fabrication de vêtements (1), en particulier de vêtements jetables pourvus d'éléments élastiques autour des ouvertures de passage des jambes, caractérisé par les opérations suivantes :
a) formation d'une bande de base (17), en amenant en continu et en collant des premiers et seconds éléments élastiques continus (4A,4B) maintenus à l'état étiré, sur une surface d'une première bande (014) en mouvement, de façon à ce que les éléments élastiques décrivent des courbes sinusoïdales, dont les sommets et les points les plus bas se présentent symétriquement, en opposition mutuelle, et qui se croisent au niveau de leurs points d'inflexion, de sorte que des zones élastiques circulaires sont formées;
b) formation d'une première bande composite (19), en sectionnant la bande de base (17) en suivant des lignes (18) passant par les points d'intersection (16') respectifs, au niveau desquels s'entrecoupent les premiers et seconds éléments élastiques (4A,4B), et en collant, ponctuellement, les diverses bandelettes sectionnées, avec de l'adhésif, sur une seconde bande (07) en mouvement, le long de sa zone médiane longitudinale;
c) formation d'une deuxième bande composite (20), en collant une troisième bande en mouvement, avec de l'adhésif, sur une surface de la première bande composite (19);
d) formation de coupes de vêtements liés les uns aux autres, par suppression, en les découpant, de zones de la deuxième bande composite (20) délimitées par les zones élastiques circulaires respectives (16), pour former des réservations (13) pour les ouvertures de passage des jambes; et
e) fabrication de vêtements individuels (1), par séparation des coupes de vêtements tenant ensemble, en suivant les lignes divisant, en deux moitiés, dans le sens longitudinal de la seconde bande composite, une zone élastique circulaire correspondante.

2. Procédé selon la revendication 1, caractérisé en ce qu'après l'opération (c) de la revendication 1, on exécute les opérations intermédiaires suivantes :
f) pliage de la seconde bande composite (20) dans le sens transversal, en suivant sa ligne médiane longitudinale (21) qui la partage en deux moitiés;
g) formation de lignes d'adhérence (23), délimitant les vêtements individuels, sur la seconde bande composite (20), en suivant des lignes (22) partageant les zones circulaires respectives en deux moitiés;
h) séparation des vêtements individuels (1) de la coupe de vêtements continue (01), simultanément à ou après l'opération (g), en sorte que les lignes d'adhérence (23) soient conservées et forment ainsi les lisières latérales (24) des vêtements individuels,
procédé selon lequel l'opération (d) de la revendication 1 peut être exécutée avant ou après l'opération (f).
